# EUROPEAN PATENT APPLICATION

(11) **EP 2 633 839 A2**
(43) Date of publication of application: **04.09.2013**
(21) Application number: 13157279.4
(22) Date of filing: 28.02.2013
(51) Int. Cl.: A61F 9/00

(54) **Balloon punctal plug**

(30) Priority: 28.02.2012 US 201213407306
(71) Applicant: Johnson & Johnson Vision Care, Inc., Jacksonville, FL 32256 (US)
(72) Inventor: Lust, Victor, Jacksonville, FL Florida 32223 (US); Miller, Jeffrey, St. Augustine, FL Florida 32086 (US); Yewey, Gerald, Jacksonville, FL Florida 32250 (US); Allen, Robert Wade, Memphis, TN Tennessee 38104 (US); Mendius, Richard W., Collierville, TN Tennessee 38017 (US)
(74) Representative: Tunstall, Christopher Stephen

(57) **Abstract**

A lacrimal insert such as a punctal plug may incorporate a balloon or other similar structure to facilitate anchoring of the punctal plug within the lacrimal canaliculus. The balloon may be inflated once it is positioned and deflated if removal is required or desired. The balloon may be affixed to an external portion of the punctal plug or incorporated into a chamber within the punctal plug.

## Description

### BACKGROUND OF THE INVENTION

### 1. Field of the Invention

The present invention relates to a lacrimal insert, and more particularly to a lacrimal insert such as a punctal plug comprising an inflatable and deflatable structure such as a balloon to anchor the punctal plug in the lacrimal canaliculus.

### 2. Discussion of the Related Art

Insufficient tears, or "dry eye" is a common condition caused by the insufficient production of tears from the lacrimal gland which causes symptoms such as dryness, redness, burning, reflex tearing, itching, or foreign body sensation. In especially difficult cases of dry eye, a lacrimal insert or punctal plug may be placed into one or both of the lacrimal puncta. Punctal plugs prevent the tears, which are being produced in deficient volume by the lacrimal gland, from draining into the lacrimal canaliculi.

The corner of each eye is called a canthus, with the nose side called the nasal canthus and the temporal side called the temporal canthus. At the lower and upper eyelid margins of the nasal canthus are small openings called puncti or puncta. As used herein, both puncti and puncta shall be understood to be the plural form of punctum. Each punctum drains tears from the eyes. A punctal plug or occluder is an ophthalmic device for insertion into a punctum of an eye in order to treat one or more disease states. Typically, a punctal plug is positioned to block tear drainage thereby helping treat dry eyes. Punctal plugs may also be utilized for sustained release of medication to the eye for the treatment of a wide variety of ocular diseases.

In order to treat infection, inflammation, glaucoma, and other ocular diseases, drugs are often required to be administered to the eye. A conventional method of drug delivery is by topical application to the eye's surface. The eye is uniquely suited to this surface route of drug administration because, properly constituted, drugs can penetrate through the cornea, rise to therapeutic concentration levels inside the eye, and exert their beneficial effects. In practice, eye drops currently account for more than ninety-five (95) percent of drug delivery methods for the eye. Rarely are drugs for the eye administered orally or by injection, either because they reach the eye in too low a concentration to have the desired pharmacological effect, or because their use is complicated by significant systemic side effects.

Eye drops, though effective, are unrefined and inefficient. When an eye drop is instilled in the eye, it typically overfills the conjuctival sac, the pocket between the eye and the eyelids, causing a substantial portion of the drop to be lost due to overflow of the eyelid margin onto the cheek. In addition, a substantial portion of the drop remaining on the ocular surface is washed away by tears into the tear drainage system, thereby diluting the concentration of the drug. Not only is this share of the drug dose lost before it can cross the cornea, but this excess drug may be carried into the nose and throat where it is absorbed into the general circulation, sometimes leading to serious systemic side effects. The small portion of the drug in the eye drop which does penetrate the cornea results in an initial peak tissue concentration, a higher level than is required for the initial pharmacological effect. This tissue concentration then gradually decreases, such that by the time the next eye drop is due, the tissue concentration and the intended pharmacological effect may be too low.

To compound the problems described above, patients often do not use their eye drops as prescribed. Often, this poor compliance is due to an initial stinging or burning sensation caused by the eye drop. Certainly, instilling eye drops in one's own eye can be difficult, in part because of the normal reflex to protect the eye. Older patients may have additional problems instilling drops due to arthritis, unsteadiness, and decreased vision, and pediatric and psychiatric patient populations pose difficulties as well. Accordingly, punctal plugs provide a viable means for solving the problems of reliable and efficient drug delivery to the eye.

Punctal plugs may be of the temporary variety or of the permanent variety. Temporary punctal plugs are usually fabricated from collagen or other similar material and are dissolvable. Temporary punctal plugs may be utilized for short duration treatment or to gauge how an individual will react to having the insert placed, for example, will the device cause excessive tearing. Permanent punctal plugs are for long term use and are removable at any time. Permanent punctal plugs are available in various sizes with the largest size that fits providing maximum effectiveness. Permanent punctal plugs are typically made of silicone rubber.

A punctal plug typically includes a body portion sized to pass through a lacrimal punctum and be positioned within a lacrimal canaliculus of the eyelid. The punctal plug also comprises a collarette connected to the body portion and sized to rest on the exterior of the lacrimal punctum. The term lacrimal punctum and lacrimal canaliculus are often utilized interchangeably; however, as used herein, the punctum means the opening and the canaliculus is the passageway or duct-like pathways that lead to the lacrimal sac. If the punctal plug is used to deliver therapeutic agents to the eye, then the body portion may comprise a reservoir for holding the therapeutic agents and the collarette may comprise an opening in communication with the reservoir through which the therapeutic agents are released.

The anatomy of the drainage system of the human eye may vary slightly from one individual to another and may vary from one eye to another. Accordingly, punctal plugs may be manufactured in a number of sizes. However, regardless of the number of different sizes available, no one size fits all. In addition, a reduction in the profile of the device for ease of insertion and removal while maintaining a secure fit would be beneficial. Accordingly, there exists a need for a punctal plug with a means for improved placement and securement in varied anatomies.

### SUMMARY OF THE INVENTION

The punctal plug incorporating a balloon structure in accordance with the present invention overcomes the limitations associated with the prior art devices as briefly described above.

In accordance with a first aspect, the present invention is directed to a lacrimal insert. The lacrimal insert comprising a punctal plug including a body portion having first and second ends, and an inflatable/deflatable element cooperatively associated with the first end of the body portion.

In accordance with another aspect, the present invention is directed to a method for treating an eye of a patient. The method comprising implanting a punctal plug, having a body portion with a first end and a second end, into a punctum of an eyelid, and inflating an inflatable/deflatable element cooperatively associated with the first end of the body portion to anchor the punctal plug in a lacrimal canaliculus.

The present invention is directed to a lacrimal insert or punctal plug incorporating a balloon structure that may be inflated and deflated multiple times. The balloon structure may be utilized to secure the punctal plug in position. In other words, the balloon punctal plug of the present invention may be inserted into either the upper and/or lower lacrimal canaliculus with the balloon deflated and then secured in position by inflating the balloon. If and/or when the device has to be removed, the balloon may be deflated. Although a balloon is described herein, any similar structure may be utilized.

The inflatable/deflatable balloon punctal plug may be inserted into either or both the upper or lower lacrimal canaliculus. The balloon punctal plug is inserted into the punctum and then inflated by any suitable means, including the use of saline. When the punctal plug has been inserted into the lacrimal duct and the lower head inflated via the balloon, this then causes the head to expand and conform to the anatomy of the vertical/horizontal canaliculus of the patient. This one size fits all device may be utilized for a majority of patients requiring this type of treatment. The insertion forces are negligible due to the low profile of the non-expanded head. With the appropriate selection of material for the balloon punctal plug, the canaliculus will self-seal once the balloon is expanded via inflation.

The balloon punctal plug of the present invention provides a device capable of conforming to different anatomies without any significant increase in cost or ease of manufacture. The balloon punctal plug may also deliver a therapeutic agent similarly to existing non-balloon punctal plugs. With the inflatable or expandable balloon, the punctal plug may be more securely positioned with lower risk of accidental removal.

### BRIEF DESCRIPTION OF THE DRAWINGS

The foregoing and other features and advantages of the invention will be apparent from the following, more particular description of preferred embodiments of the invention, as illustrated in the accompanying drawings.
Figure 1 illustrates the anatomy of the lacrimal drainage system of the human eye.
Figure 2 illustrates an example of a conventional punctal plug that is known in the art.
Figure 3 illustrates an example of a punctal plug, including a reservoir for the release of one or more therapeutic agents that is known in the art.
Figures 4A and 4B are diagrammatic representations of a first balloon punctal plug in accordance with the present invention.
Figures 5A and 5B are diagrammatic representations of a second balloon punctal plug in accordance with the present invention.
Figures 6A and 6B are diagrammatic representations of a third balloon punctal plug in accordance with the present invention.
Figures 7A and 7B are diagrammatic representations of a fourth balloon punctal plug in accordance with the present invention.
Figures 8A and 8B are diagrammatic representations of a fifth balloon punctal plug in accordance with the present invention.

### DETAILED DESCRIPTION OF THE PREFERRED EMBODIMENTS

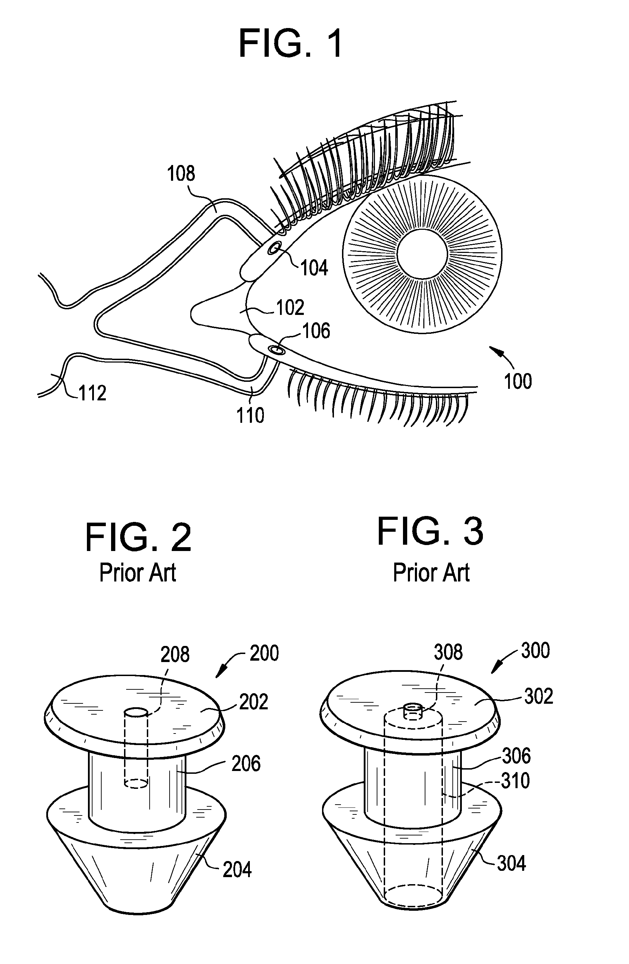

Figure 1 illustrates the anatomy of the drainage system of a human eye 100. Tears are produced by the lacrimal gland, not illustrated, superior to the outer portion of each eye 100. Tears flow across the surface of the eye 100 to a shallow pool, termed the lacrimal lake 102, located where the upper and lower eyelids come together at their inner ends or nasal ends. From there, the tears drain through small openings in each of the eyelids, namely, the upper lacrimal punctum 104 and the lower lacrimal punctum 106. From the upper lacrimal punctum 104 and the lower lacrimal punctum 106, the tears pass into the upper lacrimal canaliculus 108 and lower lacrimal canaliculus 110, respectively, which are duct-like pathways leading to the lacrimal sac 112. The lacrimal sac 112 is the superior, expanded portion of the nasolacrimal duct, not illustrated, which drains tears into the nasal system. The upper lacrimal punctum 104 and associated canaliculus 108 typically only drain about ten percent of the tears from the eye 100, such that their obstruction virtually never leads to the tear overflow.

Tears or the tear film comprises three layers. The first layer or bottom layer is the layer that coats the eye and comprises mucin which is created by cells in the conjunctiva referred to as goblet cells. The mucin fills in microscopic irregularities on or in the eye's surface which is important to clear vision. The second layer or middle layer of the tear film comprises essentially water and makes up the bulk of the tear film. A majority of the watery component is produced or supplied from the main lacrimal or tear gland. Emotional tears and reflect tears, i.e. tears resulting from a stimulus such as bright light or a foreign body, come from the main lacrimal gland. Accessory lacrimal glands, known as the glands of Wolfing and Kraus are found in the eyelid tissue and also contribute to the watery component. The third or top layer of the tear film comprises a thin layer of oil secreted by the meibomian glands and functions to prevent the tears from evaporating too quickly.

Insufficient tears, or "dry eye" is a common condition caused by insufficient production of tears from the lacrimal gland which causes symptoms such as dryness, redness, burning, reflex tearing, itching, or foreign body sensation. In especially difficult cases of dry eye, a punctal occluder or punctal plug may be placed into one or both of the lacrimal puncta 104, 106, see Figure 1. Punctal plugs prevent the tears, which are being produced in deficient volume by the lacrimal gland, from draining into the lacrimal canaliculi 108, 110. Punctal plugs may be secured in the lacrimal puncta without anesthesia and removed with ease when required.

Referring now to Figure 2, there is illustrated an exemplary punctal plug 200. The punctal occluder or plug 200 comprises a collarette 202 which is configured to rest on the exterior of the punctum 104, 106 (Figure 1), a bulb 204 that blockingly projects into the canaliculus 108, 110 (Figure 1), and a body portion 206 connecting the collarette 202 and the bulb 204. Commercially available punctal plugs usually have a length of approximately 2.0 millimeters, and differ from each other only slightly in configuration. For example, the bulbs of the punctal plugs are designed to prevent the plug from being easily dislodged from the canaliculus, and may be tapered for ease of insertion into the puncta. The collerette is designed to have a diameter sufficient to prevent the plug from completely entering the canaliculus, and are preferably smooth to minimize irritation of the eye. The body portions of different punctal plugs are also similar in design and are essentially a non-functional connection between the collarette and the bulb portions. The collarette 202 may include an aperture 208, illustrated in phantom, extending into the body portion 206 to aid in grasping or securing the punctal plug 200 during its insertion into the puncta. Examples of punctal plugs may be found in U.S. Patent Number 3,949,750 and 5,283,063 to Freeman, U.S. Patent Numbers 5,053,030, 5,171,270 and 5,723,005 to Herrick, U.S. Patent Number 5,417,651 to Guena et al. and U.S. Patent Number 5,423,777 to Tajiri et al.

In addition to, or alternately, a punctal occluder or plug may be utilized to deliver one or more therapeutic agents and/or medications. Figure 3 illustrates an ophthalmic insert or punctal plug 300 that adapts the form of a conventional punctal plug 200, as illustrated in Figure 2, to incorporate a reservoir 310, illustrated in phantom, designed to store and release medication onto the surface of the eye. The reservoir 310 may be configured to release the medication in any number of ways, including pulsatile and continuous. In addition, the reservoir may be refilled as required. The ophthalmic insert or punctal plug 300 comprises a collarette 302, a bulb 304 and a body portion 306. The punctal plug 300 may be molded or otherwise formed from a flexible material, such as silicone, that is impermeable to the medication which will fill the reservoir 310. Although silicone is described herein, it is important to note that any suitable biocompatible material may be utilized. The reservoir 310 may be formed by a channel through the interior of the body portion 306 of the plug 300. The body portion 306 may be flexible, or even accordion shape so as to provide the capability of lengthwise expansion as it is filled with medication. The collarette 302 anchors the plug 300 to the exterior of the lacrimal punctum 104 and 106 (see Figure 1) and may be provided with an opening 308 which is in fluid communication with the reservoir 310. In order to control the delivery of a specific medication, the geometry of the opening 308 may be customized in a variety of ways. For example, the opening 308 may be designed for releasing the medication at a constant sustained release rate, a pulsatile release rate, an exponential release rate and/or any combination thereof. Through opening 308, medication is released from the reservoir 310 into the tears of the lacrimal lake where the medication mixes, as eye drops do, with the tears and penetrate the eye to have the intended pharmacological and therapeutic effect. Although not required, the punctal plug 300 may comprise an enlarged bulb 304 to help secure the plug 300 in position within the canaliculus and also to provide additional volume for the reservoir as illustrated. An exemplary device may be found in U.S. Patent Number 6,196,993 to Cohan et al.

Punctal plugs may take on any number of configurations, sizes and be formed from any number of materials, depending on the desired functionality and/or medications to be delivered.

As set forth above, punctal plugs may take any size and shape. Typically, the body of the punctal plug is in the shape of an elongated cylinder, and may vary in length in the range from about 0.8 mm to about 5 mm and may vary in width in the range from about 0.2 mm to about 3 mm. The size of the opening for medication or drug release may be in the range from about 1 nm to about 2.5 mm. Rather than one large opening at any one location, multiple small openings may be used. The body of the punctal plug may be wholly or partially transparent or opaque. Optionally, the body may include a tint or pigment that makes the plug easier to see when it is placed in a punctum.

Punctal plugs may be fabricated from any number of suitable biocompatible materials including silicone, silicone blends, silicone copolymers, for example, hydrophilic monomers of polyhdroxyethlmethacrylate, polyethylene glycol, polyvinylpyrrolidone and glycerol, and silicone hydrogel polymers, for example, those described in U.S. Patent Nos. 5,962,548, 6,020,445, 6,099,852, 6,367,929, and 6,822,016. Other suitable biocompatible materials include polyurethane, polymethylmethacrylate, poly(ethylene glycol), poly(ethylene oxide), poly(propylene glycol), poly(vinyl alcohol), poly(hydroxyethylmethacrylate), poly(vinylpyrrolidone), polyarcrylic, poly(ethyloxazoline), poly(dimethyl acrylamide), phospholipids, for example, phosphoryl choline derivatives, polysulfobetains, acrylic esters, polysaccharides and carbohydrates, for example, hyaluronic acid, dextran, hydroxyethyl cellulose, hydroxyl propyl cellulose, gellan gum, guar gum, heparin sulfate, chondroitin sulfate, heparin and alginate, proteins, for example, gelatin, collagen, albumin and ovalbunin, polyamino acids, fluorinated polymers, for example, polytetrafluoroethylene and polyvinylidine fluoride, polypropylene, polyethylene, nylon and ethylene-co-vinylacetate.

The exterior surfaces of the punctal plug may be wholly or partially coated with a number of different biocompatible coatings. The coating may provide a number of benefits, including lubriciousness to aid in insertion of the device, muco-adhesiveness to improve tissue compatibility, texture to aid in anchoring the device and/or any combination thereof. Suitable biocompatible coatings include gelatin, collagen, hydroxyethyl methacrylate, poly(vinylpyrrolidone), poly(ethylene glycol), heparin, chondroitin sulfate, hyaluronic acid, synthetic and natural proteins, polysaccharides, thiomens, thiolated derivates of polyacrylic acid and chitosan, polyacrylic acid, carboxymethal cellulose and combinations thereof.

It has been found that with certain therapeutic agents or medications, it may be desirable to create a barrier layer between the therapeutic agent containing material to be released from the reservoir within the punctal plug and the interior surface of the walls that define the reservoir due to possible interactions, or inadvertent leaching of the active therapeutic agent through the wall of the punctal plug. In addition, it has been found that the retention of therapeutic agent within the reservoir may be aided by the selection of the geometric configuration of the punctal plug, or with the addition of various anchoring features. For example, a reservoir may comprise a simple cylindrical configuration which may not securely hold a particular therapeutic agent within the reservoir. In other words, that shape, even with a primer layer or adhesive layer may not be sufficient to hold the agent in place. Accordingly, the geometry of the reservoir may be modified to include protrusions or indents for holding the agent. These geometric variations may be utilized alone or in combination with various barrier layers, adhesives and/or primer layers. In other words, various combinations of geometries and coatings may be utilized to hold the drug in and/or force the drug out as required. For example, a barrier layer may be disposed on the external surface of the punctal plug to inhibit diffusion of the therapeutic agent in the body of the punctal plug and to inhibit the infusion of tears into the reservoir containing the therapeutic agent. In addition, the geometry of the punctal plug may be modified to create a better fit within the canaliculus.

The present invention is directed to a lacrimal insert or punctal plug incorporating a balloon structure that may be inflated and deflated multiple times. The balloon structure may be utilized to secure the punctal plug in position within the lacrimal canaliculus. In other words, the balloon punctal plug of the present invention may be inserted into either the upper or lower lacrimal canaliculus with the balloon deflated and then secured in position by inflating the balloon. If and when the punctal plug has to be removed, the balloon may be simply deflated and the low profile device removed. Although a balloon is shown and described herein, any similar structure may be utilized.

As set forth above, the inflatable/deflatable balloon punctal plug may be inserted into either the upper or lower lacrimal canaliculus. The balloon punctal plug is inserted into the punctam and then inflated by any suitable means, including saline solution, air or a medium viscosity fluid, e.g. up to 5000 cp. When the punctal plug has been inserted into the lacrimal duct and the lower head inflated, this then causes the head to expand and conform to the anatomy of the vertical/horizontal canaliculs of the patient. This one size fits all device may be utilized for a majority of patients requiring this type of treatment. The insertion forces are negligible due to the collapse of head, for example, there is no fixed or static arrowhead as illustrated in Figures 2 and 3. With the appropriate selection of material for the balloon punctal plug, the canaliculus will self-seal once the balloon is expanded via inflation.

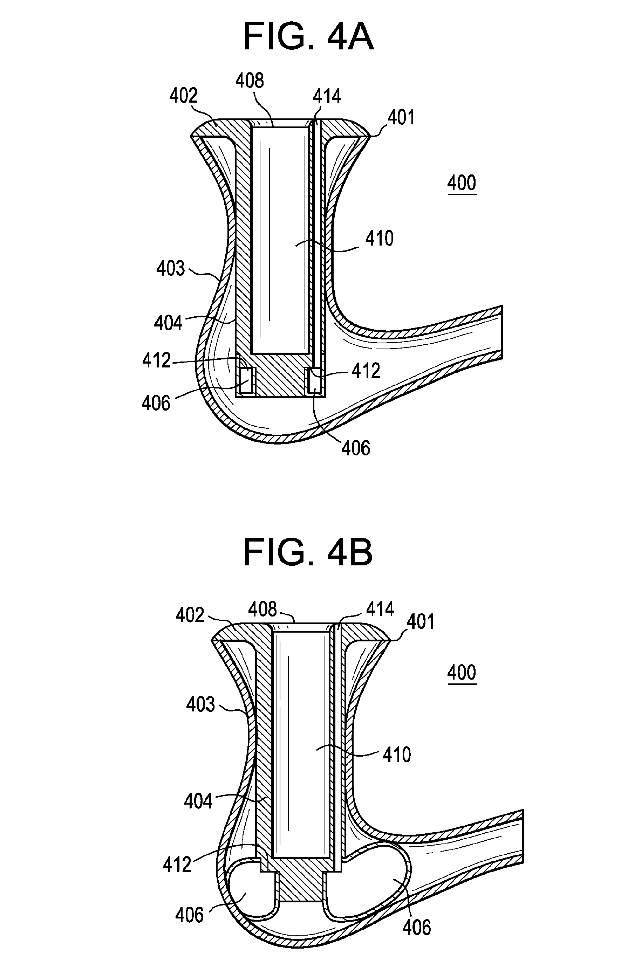

Referring now to Figures 4A and 4B, there is illustrated a balloon punctal plug 400 in accordance with the present invention. The balloon punctal plug 400 comprises a collarette 402 which is configured to rest on the exterior of the punctum 401, a body portion 404 extending from the collarette 402 and which projects into the lacrimal canaliculus 403, and a balloon head 406. The collarette 402 may include an aperture 408 extending into the body portion 404 to aid in grasping or securing the balloon punctal plug 400 for insertion and /or removal. The aperture 408 may incorporate a reservoir 410 that is configured to release a therapeutic agent or medication as described above. The balloon head 406 may be positioned and affixed to a shoulder region 412 formed in the end of the body 404 opposite or distal relative to the end connected to collarette 402. The balloon head 406 may be formed from any suitable material that may be easily inflated and deflated as well as conform to the shape of the lacrimal canaliculus 403. The balloon head 406 may be formed from nylon. It is important to note; however, that any suitable biocompatible material may be utilized. The wall thickness of the balloon head 406 may be varied from region to region around the body portion 404 in order to achieve a better fit. For example, a thinner region will expand more than a thicker region and thus the shape of the balloon 406 may be tailored to fit different anatomical structures. An inflation port 414 may be incorporated into any suitable location on the balloon punctal plug 400. As illustratedin Figures 4A and 4B, the inflation port 414 may extend from the collarette 402 along the length of the body 404 and into fluid communication with the balloon head 406. As stated above, the balloon head 406 may be inflated with any suitable material, preferably, a material that is biocompatible, such as saline. As illustrated in Figure 4B, once the balloon head 406 is inflated it may assume a configuration that allows the balloon punctal plug 400 to be sealed within the lacrimal canaliculus 403. In other words, the balloon head substantially assumes the shape or configuration of the lacrimal canaliculus when inflated, but while deflated or unexpanded, the balloon punctal plug may be easily inserted into the lacrimal canaliculus. For removal, the balloon head 406 is simply deflated thereby once again reducing the overall profile of the balloon punctal plug 400.

The balloon head or balloon 406 may be affixed to the body 404 at the shoulder region 412 by any suitable means including adhesives and welding. As illustrated in Figures 4A and 4B, the balloon may be is external to the plug 400 and it is preferable that it not separate therefrom.

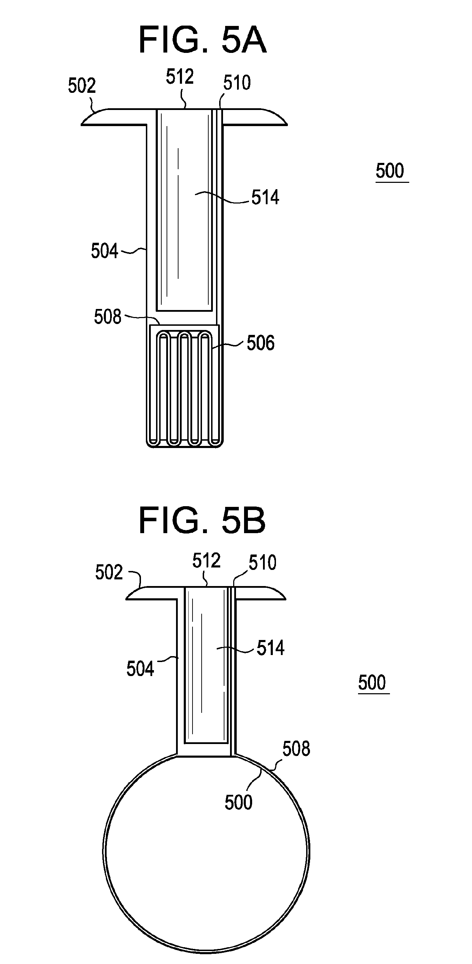

As illustrated in Figures 4A and 4B, the balloon head 406 may comprise a uniform thickness and may be affixed externally to the body 404. Alternatively, the balloon head or balloon may be internally mounted and comprise alternate configurations. Figures 5A and 5B illustrate a balloon punctal plug 500 in accordance with the present invention. The balloon punctal plug 500 comprises a collarette 502 which is configured to rest on the exterior of the punctum, not illustrated, a body portion 504 extending from the collarette 502 and which projects into the lacrimal canaliculus, not illustrated, a balloon 506 positioned in a balloon chamber 508 and a balloon inflation lumen 510 which extends along the length of the balloon punctal plug 500 from the collarette 502 to the balloon 506. The collarette 502 may include an aperture 512 extending into the body portion 504 to aid in grasping or securing the balloon punctal plug 500 during insertion and/or removal. The aperture 512 may incorporate a reservoir 514 that is configured to release a therapeutic agent or medication as discussed above. Once again, the inflation lumen 510 may be positioned at any suitable location, preferably where it does not increase the profile of the balloon punctal plug 500.

As illustrated in Figures 5A and 5B, the balloon 506 may be positioned within a chamber 508 at the end of the body 504 distal from the collarette 502. The chamber 508 may comprise any suitable design and preferably is formed from a material that is suitably flexible to allow the balloon 506 to easily expand when inflated. This may be controlled by a combination of material, for example, silicone rubber, and/or wall thickness. Directional control of balloon expansion may be achieved by varying different portions of the chamber 508 as described above with respect to the thin and thick zones of the balloon. As illustrated, the balloon 506 may be folded or pleated within the balloon chamber 508 in order to ensure a minimal profile for a potentially large balloon 506. Any suitable pleating or folding configuration may be utilized. Different fold configurations may result in different inflation profiles. Figure 5B illustrates the balloon 506 in an expanded or inflated state thus creating a substantially spherical configuration. Given that the balloon 506 is within the balloon chamber 508, the balloon chamber 508 comprises substantially one same configuration.

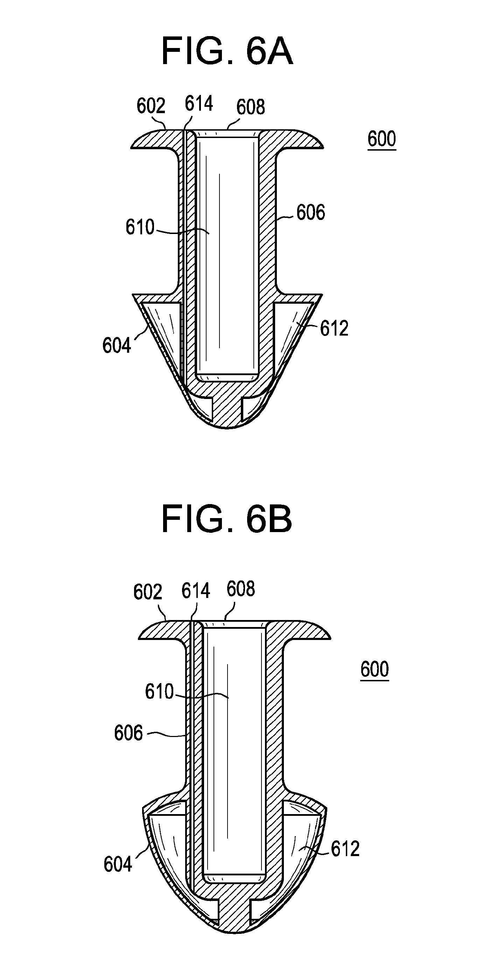

Referring now to Figures 6A and 6B, there is illustrated a third balloon punctal plug 600 in accordance with the present invention. The balloon punctal plug 600 comprises a collarette 602 which is configured to rest on the exterior of the punctum, not illustrated, a bulb 604 that blockingly projects into the lacrimal canaliculus, not illustrated, and a body portion 606 connecting the collarette 602 and the bulb 604. The punctual plug illustrated in Figures 6A and 6B is similar in outward construction to that of the prior art device illustrated in Figures 2 and 3. The collarette 602 may include an aperture 608 extending into the body portion 606 to aid in grasping or securing the punctal plug 600 during its insertion and/or removal from the puncta. The aperture 608 may also comprise a reservoir 610 as set forth with respect to the above described Figures 3A, 3B, 4A, 4B, 5A and 5B. The balloon punctal plug 600 also comprises a balloon 612 positioned within the bulb 604. A balloon inflation lumen 614 extends from the collarette 602 to the balloon 612 in the bulb 604. The inflation lumen 614 is preferably positioned so as not to increase the profile of the balloon punctal plug 600. As previously described herein the balloon 612 may comprise any suitable material and may be configured to expand in any number of directions and/or orientations. As illustrated in Figure 6B, this balloon 612 tends to round out and thicken portions of the bulb 604.

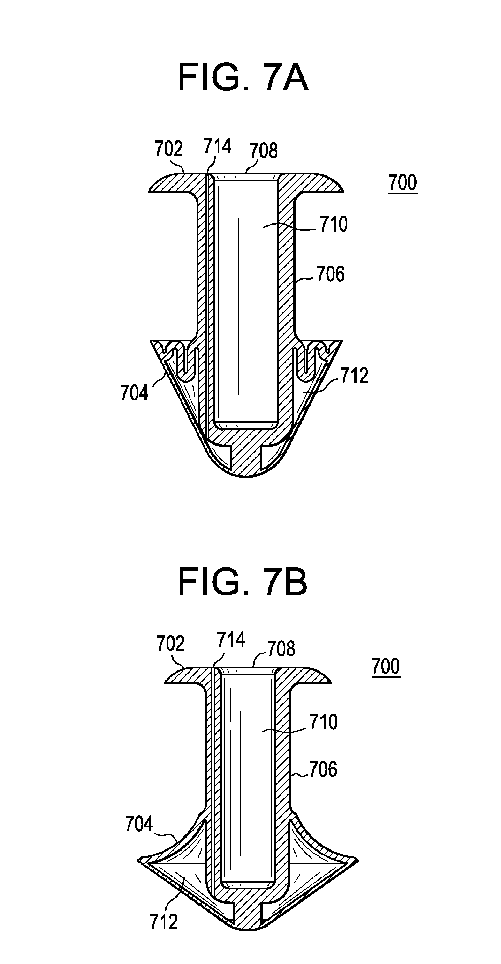

Figures 7A and 7B illustrate a fourth balloon punctal plug 700 in accordance with the present invention. The balloon punctal plug 700 comprises a collarette 702 which is configured to rest on the exterior of the punctum, not illustrated, a bulb 704 that blocking projects into the lacrimal canaliculus, not illustrated, and a body portion 706 connecting the collarette 702 and the bulb 704. The collarette 702 may include an aperture 708 extending into the body portion 706 to aid in grasping or securing the punctal plug 700 during its insertion and/or removal from the puncta. The aperture 708 may also comprise a reservoir 710 as previously described herein. The balloon punctal plug 700 also comprises a balloon 712 positioned within the bulb 704. A balloon inflation lumen 714 extends from the collarette 702 to the balloon 712 in the bulb 704. Once again, the inflation lumen 714 is preferably positioned to maintain a low profile in the balloon punctal plug 700. The balloon 712 may comprise a folded or pleated structure that expands or inflates into the shape illustrated in Figure 7B. As previously described herein, the balloon 712 may comprise any suitable material and may be configured to expand in any number of directions and/or orientations. The pleated nature of the balloon 712/bulb 704 configuration provides for an almost diamond or anchor shape to the bulb 704 when inflated as illustrated in Figure 7B.

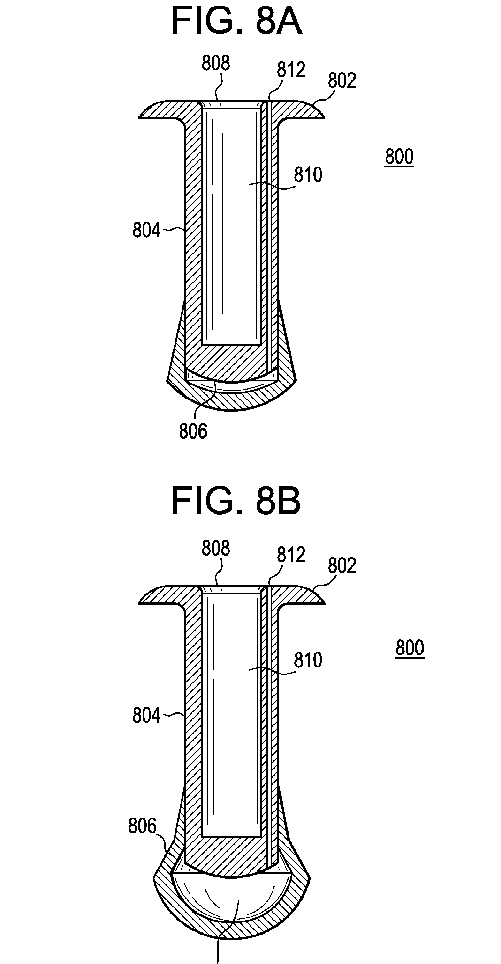

Figures 8A and 8B illustrate a fifth balloon punctal plug 800 in accordance with the present invention. As described previously in relation to Figures 4A and 4B, the balloon head 806 is once again affixed to the external portion of the balloon punctal plug 800. The balloon punctal plug 800 comprises a collarette 802 which is configured to set on the exterior of the punctum, not illustrated, a body portion 804 extending from the collarette 802 and which projects into the lacrimal canaliculus, not illustrated, and a balloon head 806. Unlike the exemplary embodiment illustrated in Figures 4A and 4B, the balloon head 806 does not sit on a shoulder region of the body 804. The collarette 802 may comprise an aperture 808 extending into the body portion 804 to aid in grasping or securing the balloon punctal plug 800 during its insertion and/or removal from the puncta. The aperture 808 may incorporate a reservoir 810 that is configured to release a therapeutic agent or medication as described above. The balloon head 806 may be affixed to a distal portion of the body 804 relative to the collarette 802. The balloon head 806 may be affixed in a suitable manner utilizing any suitable means for attachment, including welding the balloon material to the body 804 or utilizing adhesive. An inflation lumen 812 extending from the collarette 802 to the balloon head 806 may be utilized to inflate the balloon head 806 to the configuration illustrated in Figure 8B. As previously describedherein, the inflation lumen 812 is positioned to maintain the low profile of the balloon punctal plug 800.

In the punctual plugs of the present invention, the balloon may comprise various materials, thicknesses, folds and the like to create any number of anchoring configurations to secure the punctal plug in place within the lacrimal canaliculus. The balloon may be incorporated within the body or bulb/head of the punctal plug or affixed to an external portion thereof.

Aspects of the invention include:
Aspect 1. A lacrimal insert comprising:
   a punctal plug including a body portion having first and second ends; and
   an inflatable/deflatable element cooperatively associated with the first end of the body portion.
Aspect 2. The lacrimal insert according to aspect 1, further comprising a collarette configured to rest on the exterior of a punctum, the collarette being affixed to the second end of the body portion.
Aspect 3. The lacrimal insert according to aspect 2, wherein the collarette comprises an aperture.
Aspect 4. The lacrimal insert according to aspect 3, wherein the body portion comprises a reservoir.
Aspect 5. The lacrimal insert according to aspect 1, wherein the punctal plug is fabricated from a thermoset elastomer.
Aspect 6. The lacrimal insert according to aspect 5, wherein the thermoset elastomer comprises silicone.
Aspect 7. The lacrimal insert according to aspect 2, wherein the inflatable/deflatable element is affixed to an exterior portion of the body portion proximate the first end.
Aspect 8. The lacrimal insert according to aspect 2, wherein the first end of the body portion includes a compartment.
Aspect 9. The lacrimal insert according to aspect 8, wherein the inflatable/deflatable element is contained within the compartment
Aspect 10. The lacrimal insert according to aspect 2, wherein the first end of the body portion comprises a bulb.
Aspect 11. The lacrimal insert according to aspect 10, wherein the inflatable/deflatable element is contained within the bulb.
Aspect 12. The lacrimal insert according to aspect 7, wherein the inflatable/deflatable element comprises a balloon structure.
Aspect 13. The lacrimal insert according to aspect 12, further comprising an inflation port extending from the collarette to the balloon structure.
Aspect 14. The lacrimal insert according to aspect 9, wherein the inflatable/deflatable element comprises a balloon structure.
Aspect 15. The lacrimal insert according to aspect 14, further comprises an inflation port extending from the collarette to the balloon structure.
Aspect 16. The lacrimal insert according to aspect 11, wherein the inflatable/deflatable element comprises a balloon structure.
Aspect 17. The lacrimal insert according to aspect 16, further comprising an inflation port extending from the collarette to the balloon structure.
Aspect 18. A method for treating an eye of a patient, the method comprising:
   implanting a punctal plug, having a body portion with a first end and a second end, into a punctum of an eyelid; and
   inflating an inflatable/deflatable element cooperatively associated with the first end of the body portion to anchor the punctal plug in a lacrimal canaliculus.

Although shown and described is what is believed to be the most practical and preferred embodiments, it is apparent that departures from specific designs and methods described and shown will suggest themselves to those skilled in the art and may be used without departing from the spirit and scope of the invention. The present invention is not restricted to the particular constructions described and illustrated, but should be constructed to cohere with all modifications that may fall within the scope of the appended claims.

## Claims

1. A lacrimal insert comprising:
a punctal plug including a body portion having first and second ends; and
an inflatable/deflatable element cooperatively associated with the first end of the body portion.

2. The lacrimal insert according to Claim 1, further comprising a collarette configured to rest on the exterior of a punctum, the collarette being affixed to the second end of the body portion.

3. The lacrimal insert according to Claim 2, wherein the collarette comprises an aperture.

4. The lacrimal insert according to any of claims 1 to 4, wherein the body portion comprises a reservoir.

5. The lacrimal insert according to any of claims 1 to 4, wherein the punctal plug is fabricated from a thermoset elastomer.

6. The lacrimal insert according to Claim 5, wherein the thermoset elastomer comprises silicone.

7. The lacrimal insert according to any of the preceding claims, wherein the inflatable/deflatable element is affixed to an exterior portion of the body portion proximate the first end.

8. The lacrimal insert according to any of the preceding claims, wherein the first end of the body portion includes a compartment.

9. The lacrimal insert according to Claim 8, wherein the inflatable/deflatable element is contained within the compartment

10. The lacrimal insert according to any of the preceding claims, wherein the first end of the body portion comprises a bulb.

11. The lacrimal insert according to Claim 10, wherein the inflatable/deflatable element is contained within the bulb.

12. The lacrimal insert according to any of the preceding claims, wherein the inflatable/deflatable element comprises a balloon structure.

13. The lacrimal insert according to Claim 12, further comprising an inflation port extending from the collarette to the balloon structure.

14. A method for treating an eye of a patient, the method comprising:
implanting a punctal plug, having a body portion with a first end and a second end, into a punctum of an eyelid; and
inflating an inflatable/deflatable element cooperatively associated with the first end of the body portion to anchor the punctal plug in a lacrimal canaliculus.
